# EUROPEAN PATENT SPECIFICATION

(11) **EP 3 920 954 B1**
(45) Date of publication and mention of the grant of the patent: **07.06.2023**
(21) Application number: 20706848.7
(22) Date of filing: 05.02.2020
(51) Int. Cl.: A61K 38/08, A61P 31/10

(54) **ANTIMICROBIAL PEPTIDE FOR ONYCHOMYCOSIS**
ANTIMIKROBIELLES PEPTID FÜR ONYCHOMYKOSE
PEPTIDE ANTIMICROBIEN POUR L'ONYCHOMYCOSE

(30) Priority: 06.02.2019 NL 2022520
(43) Date of publication of application: 15.12.2021
(73) Proprietor: CBMR Scientific Nanoscience B.V., 6686 AE Doornenburg (NL)
(72) Inventor: BROUWER, Cornelis Peter Johannes Maria, 3962 KX Wijk bij Duurstede (NL); BROERTJES, Paul, 6686 AE Doornenburg (NL); BOEKHOUT, Teunis, 1015 JR Amsterdam (NL)
(74) Representative: Verhees, Godefridus Josephus Maria
(86) International application number: PCT/NL2020/050064
(87) International publication number: WO 2020/162749

(56) References cited:
- EP-A1- 2 030 980
- DONALD E. FRY: "Antimicrobial Peptides", SURGICAL INFECTIONS, vol. 19, no. 8, 1 December 2018 (2018-12-01), pages 804-811, XP055621203, US ISSN: 1096-2964, DOI: 10.1089/sur.2018.194

## Description

### TECHNICAL FIELD OF THE INVENTION

The invention relates to the treatment of fungal nail infection (onychomycosis) with an antimicrobial peptide. The invention further relates to an antimicrobial peptide for use in the treatment of onychomycosis and to a pharmaceutical composition comprising such antimicrobial peptide for use in the treatment of onychomycosis.

### BACKGROUND OF THE INVENTION

Onychomycosis is the term used for fungal infections of the nail. A recent review of population based studies in Europe and the US found a mean prevalence of 4.3%. Onychomycosis is commonly caused by infection with dermatophytes, in particular by infection with *Microsporum, Epidermophyton,* and *Trichophyton,* wherein about 90% of cases are related to *Trichophyton rubrum.* Onychomycosis can also be caused by non-dermatophyte moulds and by yeast, such as *Candida albicans.* Onychomycosis is not only a cosmetic problem, but can have an impact on patients' quality of life. The disease can affect tactile function of the fingers or interfere with walking or exercise when toe nails are affected.

Onychomycosis is the most common disease of the nails and constitutes about a half of all nail abnormalities. Factors which contribute to these infections could be: increasing age, male sex, repeated nail damage, genetic predispositions and underlying conditions, such as diabetes, immunodeficiency or peripheral arterial disease may predispose to develop onychomycosis. It is also suggested that abnormalities in nail morphology are the predisposing factors to onychomycosis. Psoriasis is one of the most common reasons of disturbed nail morphology and the spectrum of nail changes in psoriasis is very wide. This, suggest that dystrophic nails in psoriatic patients lose their natural preventing barrier and, therefore, are more predisposed to fungal infections.

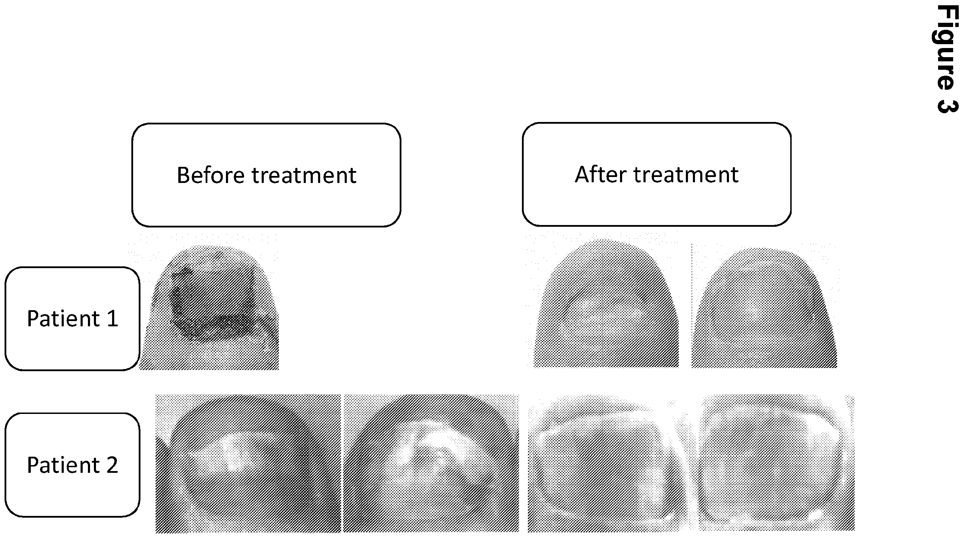

Onychomycosis may affect a single nail or multiple nails, wherein infection of toe nails is more common than finger nails. A clinical hallmark of onychomycosis is that the nail becomes friable and characteristic spikes are often visible (see Figure 3). To exclude other causes (such as for instance psoriasis, eczema, yellow nail syndrome), microscopy and culture can be performed.

Typically, topical treatment is indicated when up to 50% of the distal nail plate is involved, no more than three or four nails are affected, and for early distal and lateral subungual onychomycosis and superficial white onychomycosis.

### Available topical treatments

In many countries various topical formulations based on cyclopirix, azoles or terbinafine can be obtained over the counter at the drugstore, without any interference of a clinician/dermatologist. Effectivity is rather low (<10%), and the treatment must be continued for a very long period (*e.g*. one year).

Amorolfine 5% lacquer has a broad spectrum fungicidal and fungistatic activity and is recommended for onychomycosis without matrix involvement and for mild cases of distal and latereal disease of up to two affected nails. Application for up to 12 months results in complete cure in 12.7% of patients. Ciclopirox 8% lacquer has broad spectrum antifungal activity and leads to a complete cure in 5.5 - 8.4% of patients after 48 weeks of treatment of toe nails. Topical treatments in a specialist setting, such as tioconazole (28%) and efinaconazole (10%) show cure rates of 22% and 15.2 - 17.8%, respectively after 48 weeks of treatment. Plant extracts, such as tee tree oils are not effective (patients who are using > 5 years of tee tree oil use did not show any improvement).

Laser treatment results can be expected mostly after more sessions and takes months for improvements will be visible. Besides high costs, effectivity after 24 weeks treatment is approximately 64%.

### Available systemic treatments

Oral medication can be obtained via *e.g.* practitioners/dermatologist, but such oral medications also suffer from poor effectivity (50-70%) and severe side effects that require monitoring of liver functions. Itraconazole, terbinafine, fluconazole, and griseofulvin are used systemically to combat onychomycosis. Although up to 55% (mycological) cure have been reached with itraconazole, and 70,9% with terbinafine, systemic antifungal therapy presents with more adverse effect and interactions than topical ones.

### Available surgical treatments

Conventional therapy of onychomycosis is prolonged and often frustrating, which is why combination therapy involving topical, oral and surgical measures has been advocated as the treatment of choice. Results obtained with surgical intervention: even after removal of the entire toenail and treatment with azoles, the infections can come back. Risk of wrong ingrowth of the nail can cause a further medical problem. Surgical nail avulsion followed by topical antifungal therapy is not generally recommended for the treatment of onychomycosis.

European patent application EP2030980A1 relates to a polypeptide comprising a mutant of the amino acid sequence RRRRSVQWC, which mutation comprising polypeptide has comparable antimicrobial activity against at least one micro-organism if compared to a reference polypeptide comprising the amino acid sequence RRRRSVQWC. D.E. Fry in "Antimicrobial Peptides" (Surgical infections, V19, No.8, 2018) describes the compound norexatin for use in the treatment of onychomycosis.

### SUMMARY OF THE INVENTION

The current treatment options for onychomycosis have several drawbacks and are not very effective. Thus there is a need for an improved therapy option for onychomycosis.

The current invention provides an improved therapy option for onychomycosis in that, in one aspect, it provides an antimicrobial peptide for use in a method of preventing or treating onychomycosis, wherein the antimicrobial peptide comprises or consists of the amino acid sequence RRRRSVQWCA or ACWQVSRRRR, or a functional mutant thereof. Such antimicrobial peptide has been shown to be very effective in combating onychomycosis, according to the invention. The peptide further shows little, if any, toxicity or other side-effects. Preferably, the antimicrobial peptide is Gly Arg Arg Arg Arg Ser Val Gln Trp Cys Ala [SEQ ID NO: 3], or a functional mutant thereof.

In a second aspect, the invention provides a composition comprising an antimicrobial peptide and a pharmaceutically acceptable diluent or excipient, for use in a method of preventing or treating onychomycosis, wherein the antimicrobial peptide comprises or consists of the amino acid sequence RRRRSVQWCA or ACWQVSRRRR, or a functional mutant thereof. Such composition is preferably odourless and colourless in order to improve patients' compliance. Preferably, the antimicrobial peptide is Gly Arg Arg Arg Arg Ser Val Gln Trp Cys Ala [SEQ ID NO: 3], or a functional mutant thereof.

In a third aspect, the invention provides a method of treating a patient suffering from onychomycosis, the method comprising administering a therapeutically effective amount of an antimicrobial peptide comprising or consisting of the sequence RRRRSVQWCA or ACWQVSRRRR, or a functional mutant thereof to the cuticle of a toe affected by onychomycosis. The invention provides a method of treating a human patient suffering from onychomycosis, the method comprising topically administering a therapeutically effective amount of an antimicrobial peptide comprising or consisting of the sequence RRRRSVQWCA or ACWQVSRRRR, or a functional mutant thereof to the cuticle of a toe affected by onychomycosis, of the human patient, wherein optionally the antimicrobial peptide is provided as a solution comprising the peptide. Preferably, the antimicrobial peptide is Gly Arg Arg Arg Arg Ser Val Gln Trp Cys Ala [SEQ ID NO: 3], or a functional mutant thereof.

An aspect of the invention relates to an antimicrobial peptide for use in a method of preventing or treating onychomycosis, wherein the antimicrobial peptide comprises or consists of the amino acid sequence RRRRSVQWCA or ACWQVSRRRR or GRRRRSVQWCA or ACWQVSRRRRG, or a functional mutant thereof. Preferably, the antimicrobial peptide is Gly Arg Arg Arg Arg Ser Val Gln Trp Cys Ala [SEQ ID NO: 3], or a functional mutant thereof.

An aspect of the invention relates to a composition comprising an antimicrobial peptide and a pharmaceutically acceptable diluent or excipient, for use in a method of preventing or treating onychomycosis, wherein the antimicrobial peptide comprises or consists of the amino acid sequence RRRRSVQWCA or ACWQVSRRRR or GRRRRSVQWCA or ACWQVSRRRRG, or a functional mutant thereof. Preferably, the antimicrobial peptide is Gly Arg Arg Arg Arg Ser Val Gln Trp Cys Ala [SEQ ID NO: 3], or a functional mutant thereof.

An embodiment is the composition for use according to the invention, wherein the antimicrobial peptide is present in a concentration of between 1 mg/l and 500 mg/l, preferably between 2 mg/l and 250 mg/l, more preferably between 3 mg/l and 150 mg/l, most preferably between 3,3 mg/l and 100 mg/l, such as 30 mg/l, 33 mg/l or 50 mg/l or 100 mg/l. An embodiment is the composition for use according to the invention, wherein the antimicrobial peptide is present in a concentration of between 0,2 mg/l and 1500 mg/l, such as 0,3 mg/l - 1200 mg/l, 0,5 mg/l - 1000 mg/l.

An embodiment is the composition for use according to the invention, wherein the antimicrobial peptide is present in a concentration between 1 mg/l and 10 mg/l, more preferably between 2 mg/l and 5 mg/l, most preferably in a concentration of about 3 mg/l or about 3,3 mg/l.

An embodiment is the composition for use according to the invention, wherein the antimicrobial peptide is present in a concentration between 10 mg/l and 100 mg/l, more preferably between 20 mg/l and 50 mg/l, most preferably in a concentration of about 30 mg/l or about 33 mg/l. Preferably, the antimicrobial peptide is Gly Arg Arg Arg Arg Ser Val Gln Trp Cys Ala [SEQ ID NO: 3], or a functional mutant thereof.

An embodiment is the antimicrobial peptide for use according to the invention or the composition for use according to the invention, wherein the peptide or composition is administered at least once weakly for at least 3 months, such as for example twice or thrice weekly or daily or twice daily for 3 months - 3 years, such as 4 months - 1 year or 6 months - 9 months.

An embodiment is the antimicrobial peptide for use according to the invention or the composition for use according to the invention, wherein the peptide or composition is administered at least once every three days.

An embodiment is the antimicrobial peptide for use according to the invention or the composition for use according to the invention, wherein the peptide or composition is administered at least once every three days, more preferably at least once every two days for at least 2 weeks.

An embodiment is the antimicrobial peptide for use according to the invention or the composition for use according to the invention, wherein the antimicrobial peptide or the composition is administered at least daily for at least two weeks, more preferably for at least 1 month, more preferably for at least 2 months, more preferably for at least 4 months, most preferably for at least 6 months, such as 6 months to 12 months or 2 weeks - 12 weeks.

An embodiment is the antimicrobial peptide for use according to the invention or the composition for use according to the invention, wherein the antimicrobial peptide or the composition is administered at least twice a day.

An embodiment is the antimicrobial peptide for use according to the invention or the composition for use according to the invention, wherein the peptide or composition is applied to the eponychium and/or to the proximal nail fold.

An embodiment is the antimicrobial peptide for use according to the invention or the composition for use according to the invention, wherein the peptide or composition is applied to the lateral nail folds and/or to the lateral nail grooves and/or to the nail plate.

An embodiment is the antimicrobial peptide for use according to the invention or the composition for use according to the invention, wherein the peptide or composition is applied to the hyponychium and/or to the proximal part of the nailbed.

An embodiment is the antimicrobial peptide for use according to the invention or the composition for use according to the invention, wherein the onychomycosis involves an infection with at least one of species of *Fusarium, Microsporum, Malassezia, Epidermophyton, Trichophyton,* and/or *Candida albicans* and/or at least one of other fungal species and/or combinations thereof. An embodiment is the antimicrobial peptide for use according to the invention or the composition for use according to the invention, wherein the onychomycosis involves an infection with at least one of *Fusarium spp, Microsporum spp, Malassezia spp, Epidermophyton spp, Trichophyton interdigitalis, Scopulariopsis brevicaulis, Hypercrealis spp* and/or *Candida albicans spp* and/or at least one of other fungal species and/or combinations thereof.

An embodiment is the antimicrobial peptide comprising the sequence RRRRSVQWCA or ACWQVSRRRR or GRRRRSVQWCA [SEQ ID NO: 3] or ACWQVSRRRRG, or a functional mutant thereof, or the antimicrobial peptide consisting of the sequence RRRRSVQWCA or ACWQVSRRRR or GRRRRSVQWCA [SEQ ID NO: 3] or ACWQVSRRRRG, for use in a method according to the invention for the treatment of a patient, preferably a human patient, suffering from onychomycosis, the method comprising or consisting of the step of administering a therapeutically effective amount of the antimicrobial peptide to the cuticle of a nail affected by the onychomycosis, of said (human) patient. An embodiment is the antimicrobial peptide for use according to the invention, wherein the antimicrobial peptide is administered at least daily as an aqueous solution comprising between 1 mg/l and 100 mg/l of the antimicrobial peptide, for at least two weeks, such as for between 2 weeks and three years, between 4 weeks and 2 years, 6 weeks - 1 year, 12 weeks - 9 months, 16 weeks - 6 months. An embodiment is the antimicrobial peptide for use according to the invention, wherein the antimicrobial peptide is administered at least twice or thrice daily as an aqueous solution comprising between 0,2 mg/l and 1500 mg/l of the antimicrobial peptide, preferably 0,3 mg/l - 1200 mg/l, 0,5 mg/l - 1000 mg/l, 1 mg/l - 500 mg/l or 2 mg/l - 250 mg/l, such as 1 mg/l - 100 mg/l, for at least one week or for at least two weeks, such as for between 2 weeks and three years, between 4 weeks and 2 years, 6 weeks - 1 year, 12 weeks - 9 months, 16 weeks - 6 months.

Disclosed the use of the antimicrobial peptide comprising or consisting of the sequence RRRRSVQWCA or ACWQVSRRRR or GRRRRSVQWCA [SEQ ID NO: 3] or ACWQVSRRRRG, or a functional mutant thereof, for the manufacture of a medicament to treat onychomycosis.

An embodiment is the antimicrobial peptide for use according to the invention or the composition for use according to the invention, wherein the antimicrobial peptide has a modified C-terminus and/or has a modified N-terminus, preferably a C-terminus that is modified by amidation and/or an N-terminus that is modified by acetylation.

The present invention is described with respect to particular embodiments but the invention is not limited thereto but only by the claims. The embodiments of the invention described herein can operate in combination and cooperation, unless specified otherwise.

### BRIEF DESCRIPTION OF THE FIGURES

The present invention will be discussed in more detail below, with reference to the attached drawings and figures, in which:
Figure 1. Schematic drawing of the anatomical structures of the nail and of the anatomical structures surrounding the nail. 1: nail; 2: nail bed; 3: germinal matrix; 4: cuticle; 5: bone (distal phalanx).
Figure 2. Ball-and-stick representation of antimicrobial peptide hLF1-11 (C₅₆H₉₅N₂₅O₁₄S) [SEQ ID NO: 3].
Figure 3. Onychomycotic nails prior and after treatment with an antimicrobial peptide [SEQ ID NO: 3] for use according to the invention. The nails were treated two times daily with 3 mg/l of the peptide, during 5-6 months.

### AMINO ACID SEQUENCES & SEQ ID NOs

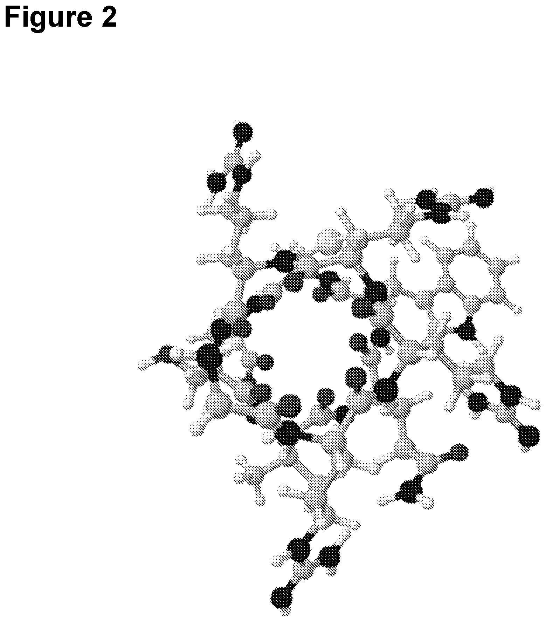

Amino acid sequence relating to human lactoferrin peptide 2-11 (hLF2-11):
   Arg Arg Arg Arg Ser Val Gln Trp Cys Ala [SEQ ID NO: 1]
Amino acid sequence relating to artificial reverse human lactoferrin peptide 2-11 (Reverse hLF2-11):
   Ala Cys Trp Gin Val Ser Arg Arg Arg Arg [SEQ ID NO: 2]
Amino acid sequence relating to human lactoferrin peptide 1-11 (hLF1-11):
   Gly Arg Arg Arg Arg Ser Val Gln Trp Cys Ala [SEQ ID NO: 3]
   See also Figure 2 for a ball and stick model of the antimicrobial peptide according to SEQ ID NO: 3.
Amino acid sequence relating to artificial reverse human lactoferrin peptide 1-11 (Reverse hLF1-11):
   Ala Cys Trp Gln Val Ser Arg Arg Arg Arg Gly [SEQ ID NO: 4]
Artificial Amino acid sequence relating to human lactoferrin peptide 1-11 provided with an N-terminal Cysteine (cysteine - hLF1-11):
   Cys Gly Arg Arg Arg Arg Ser Val Gln Trp Cys Ala [SEQ ID NO: 5]

### DEFINITIONS

The embodiments of the invention described herein can operate in combination and cooperation, unless specified otherwise.

Furthermore, the various embodiments, although referred to as "preferred" or "e.g." or "for example" or "in particular" are to be construed as exemplary manners in which the invention may be implemented rather than as limiting the scope of the invention.

The term "comprising", used in the claims, should not be interpreted as being restricted to the elements, compounds or steps listed thereafter; it does not exclude other elements or compounds or steps. It needs to be interpreted as specifying the presence of the stated features, integers, steps, compounds or components as referred to, but does not preclude the presence or addition of one or more other features, integers, steps, compounds or components, or groups thereof. Thus, the scope of the expression "a composition comprising A and B" should not be limited to a composition consisting only of compounds A and B, rather with respect to the present invention, the only enumerated compounds of the composition are A and B, and further the claim should be interpreted as including equivalents of those compounds.

### DETAILED DESCRIPTION OF THE INVENTION

In a first embodiment, the invention provides an antimicrobial peptide for use in a method of preventing or treating onychomycosis, wherein the antimicrobial peptide comprises the amino acid sequence RRRRSVQWCA or ACWQVSRRRR, or a functional mutant thereof. An aspect of the invention relates to an antimicrobial peptide for use in a method of preventing or treating onychomycosis, wherein the antimicrobial peptide comprises the amino acid sequence RRRRSVQWCA or ACWQVSRRRR, or a functional mutant thereof. An aspect of the invention relates to an antimicrobial peptide for use in a method of preventing or treating onychomycosis, wherein the antimicrobial peptide comprises the amino acid sequence RRRRSVQWCA or ACWQVSRRRR or GRRRRSVQWCA or ACWQVSRRRRG, or a functional mutant thereof. The antimicrobial peptide preferably comprises or consists of the peptide sequence GRRRRSVQWCA [SEQ ID NO: 3]. WO2009028943 describes the antimicrobial effect of antimicrobial peptides comprising the sequence RRRRSVQWCA and ACWQVSRRRR. WO2009028943 further shows mutations in said sequence, which show at least the same effect as the original sequence.

More in particular, WO2009028943 describes that the following mutations / deletions in a sequence of the invention are equally well or even better than the non-mutated sequences: at least one of the amino acids S, V, Q or W is deleted; one R and at least one of the amino acids S, V, Q or W is deleted; S, V or Q are substituted with a conservative or non-conservative amino acid, provided that (i) the substitute is not a negatively charged amino acid and (ii) S is not substituted with an N; SV, VQ or SQ are substituted with conservative or non-conservative amino acids, provided that none of the substitutes is a negatively charged amino acid; SVQ are substituted with conservative or non-conservative amino acids, provided that none of the substitutes is a negatively charged amino acid; W is replaced by another aromatic amino acid; Q or S is substituted by an aromatic amino acid and W is substituted by a neutral amino acid; C is switched with an amino acid in the SVQW sequence; at least one but no more than three of the R's are substituted by another positively charged amino acid; or the second, third or fourth R is substituted by an A.

Therefore, in a preferred embodiment, a functional mutant of the invention is chosen from the group consisting of: a deletion mutant in which at least one of the amino acids S, V, Q or W is deleted; a deletion mutant in which one R and at least one of the amino acids S, V, Q or W is deleted; a substitution mutant in which S, V or Q are substituted with a conservative or non-conservative amino acid, provided that (i) the substitute is not a negatively charged amino acid and (ii) S is not substituted with an N; a double substitution mutant in which SV, VQ or SQ are substituted with conservative or non-conservative amino acids, provided that none of the substitutes is a negatively charged amino acid; a triple substitution mutant in which SVQ are substituted with conservative or non-conservative amino acids, provided that none of the substitutes is a negatively charged amino acid; a substitution mutant in which W is replaced by another aromatic amino acid; a double substitution mutant in which Q or S is substituted by an aromatic amino acid and W is substituted by a neutral amino acid; a substitution mutant in which C is switched with an amino acid in the SVQW sequence; a substitution mutant in which at least one but no more than three of the R's are substituted by another positively charged amino acid; a substitution mutant in which the second, third or fourth R is substituted by an A; and any combination thereof. Such antimicrobial peptide may be formulated in a pharmaceutical composition. In a second embodiment, therefore, the invention provides a composition comprising an antimicrobial peptide and a pharmaceutically acceptable diluent or excipient, for use in a method of preventing or treating onychomycosis, wherein the antimicrobial peptide comprises the amino acid sequence RRRRSVQWCA or ACWQVSRRRR, or a functional mutant thereof. An aspect of the invention relates to a composition comprising an antimicrobial peptide and a pharmaceutically acceptable diluent or excipient, for use in a method of preventing or treating onychomycosis, wherein the antimicrobial peptide comprises the amino acid sequence RRRRSVQWCA or ACWQVSRRRR, or a functional mutant thereof. An aspect of the invention relates to a composition comprising an antimicrobial peptide and a pharmaceutically acceptable diluent or excipient, for use in a method of preventing or treating onychomycosis, wherein the antimicrobial peptide comprises the amino acid sequence RRRRSVQWCA or ACWQVSRRRR or GRRRRSVQWCA or ACWQVSRRRRG, or a functional mutant thereof. The antimicrobial peptide preferably comprises or consists of the peptide sequence GRRRRSVQWCA. Such composition is preferably odourless and, preferably colourless, in order to improve patients' compliance. If a composition that is to be applied to the toe or finger nails has a (nasty) odour or a colour, patients may find it difficult to apply it, for instance, before going to work, to school, or to a party. A colourless and odourless composition can be applied at any time.

An antimicrobial peptide for use according to the invention thus comprises the amino acid sequence RRRRSVQWCA or ACWQVSRRRR, or a functional mutant thereof. In a preferred embodiment, an antimicrobial peptide for use according to the invention consists of the sequence RRRRSVQWCA, GRRRRSVQWCA, ACWQVSRRRRG or ACWQVSRRRR, or a functional mutant thereof. In a preferred embodiment, the antimicrobial peptide consists of GRRRRSVQWCA. In one preferred embodiment, the peptide has been made cyclic. Various methods of cyclization are known to the skilled person, *e.g.,* Cys-Cys cyclization, backbone-cyclization (= amide bond formation), or thio-ether cyclization. Cys-Cys cyclization stems from formation of a disulfide (S-S) bond between the thiol side chains of two cysteine residues in a peptide. One disadvantage of Cys-Cys cyclized peptides is the limited stability of the S-S bond, especially under reductive conditions. Amide bond cyclization is also frequently used for cyclic peptides. The bond is chemically more stable than a Cys-Cys bond. Backbone-amide cyclized peptides are in general prepared via head-to-tail cyclization or side-chain-to-side-chain cyclization. Thio-ether cyclization is performed by reacting the side chain thiol group of a cysteine with the alpha-carbon atom of another amino acid. The reacting amino acids may already be comprised within the sequence of a peptide for use according to the invention or may be added for this specific purpose. In order for performing Cys-Cys cyclization in a peptide consisting of the sequence GRRRRSVQWCA, an additional cysteine may for instance be added. CRRRRRSVQWCA may then be cyclized by reacting the thiol groups of both cysteines present.

A peptide for use according to the invention may further be glycosylated. The skilled person is aware how to chemically glycosylate a peptide as, *e.g*., described by Crich [1]. An antimicrobial peptide for use according to the invention may, further to the RRRRSVQWCA or ACWQVSRRRR sequence, or a functional mutant thereof, comprise other amino acids, preferably positively charged amino acids, such as arginine (R) or lysine (K).

An antimicrobial peptide for use according to the invention may, further to, or in addition to the QWCKQWCK sequence, or a functional mutant thereof, comprise other amino acids, preferably positively charged amino acids, such as arginine (R).

An embodiment is the antimicrobial peptide for use in a method of preventing or treating onychomycosis according to the invention, wherein the antimicrobial peptide comprises the amino acid sequence RRRRSVQWCA or ACWQVSRRRR or GRRRRSVQWCA or ACWQVSRRRRG, or a functional mutant thereof, and wherein the C-terminus and/or the N-terminus of the antimicrobial peptide is modified. Preferably, said modified C-terminus is a C-terminus modified by amidation, and/or preferably said modified N-terminus is an acetylated N-terminus. An embodiment is the composition comprising an antimicrobial peptide and a pharmaceutically acceptable diluent or excipient, for use in a method of preventing or treating onychomycosis according to the invention, wherein the antimicrobial peptide comprises the amino acid sequence RRRRSVQWCA or ACWQVSRRRR or GRRRRSVQWCA or ACWQVSRRRRG, or a functional mutant thereof, and wherein the C-terminus and/or the N-terminus of the antimicrobial peptide is modified. Preferably, said modified C-terminus is a C-terminus modified by amidation, and/or preferably said modified N-terminus is an acetylated N-terminus. An antimicrobial peptide for use according to the invention, may, either as the sole modification(s) of the peptide, or in addition to further various modifications of the peptide and/or in addition to functional mutations of the peptide, consist of an antimicrobial peptide having a modified C-terminus, such as modification by C-terminal amidation, and/or having a modified N-terminus, such as an acetylated N-terminus. An embodiment is the antimicrobial peptide for use according to the invention, wherein the peptide comprises a modified C-terminus, and/or a modified N-terminus. An embodiment is the antimicrobial peptide for use according to the invention, wherein the antimicrobial peptide comprises a C-terminus which is amidated, and/or an N-terminus which is acetylated. The inventors found that the N-acetylated antimicrobial peptide GRRRRSVQWCA, the C-amidated antimicrobial peptide GRRRRSVQWCA, and the antimicrobial peptide GRRRRSVQWCA with both an N-acetylated terminus and a C-amidated terminus, displayed comparable or the same *in vitro* anti-microbial activity towards a series of microbial organisms (See Table 4 in the Examples section).

An embodiment is the composition for use according to the invention, wherein the antimicrobial peptide is present in a concentration of between 1 g/l and 100 g/l, preferably between 2 g/l and 50 g/l. In a preferred embodiment, a composition for use according to the invention is provided, wherein the antimicrobial peptide is present in a concentration of between 1 mg/l and 100 mg/l, preferably between 2 mg/l and 50 mg/l. An embodiment is the composition for use according to the invention, wherein the antimicrobial peptide is present in a concentration of between 1 mg/l and 500 mg/l, preferably between 2 mg/l and 250 mg/l, more preferably between 3 mg/l and 150 mg/l, most preferably between 3,3 mg/l and 100 mg/l, such as 33 mg/l or 50 mg/l.

The composition for use according to the invention is preferably provided as either a "high concentration therapy" or a "low concentration therapy", or both. With high concentration therapy and low concentration therapy is meant that the antimicrobial peptide is present in the composition for use according to the invention in a concentration of about 30 mg/l or of about 3 mg/l, respectively.

The composition is preferably provided as a " very high concentration therapy". With the term "very high concentration therapy" is meant that the antimicrobial peptide is present in the composition for use according to the invention in a concentration of about 100 mg/l or higher.

An embodiment is the composition for use according to the invention, wherein the antimicrobial peptide is present in a concentration between 1 g/l and 10 g/l, more preferably between 2 g/l and 5 g/l, most preferably of about 3 g/l. In one preferred embodiment, therefore, a composition for use according to the invention is provided, wherein the antimicrobial peptide is present in a concentration between 1 mg/l and 10 mg/l, more preferably between 2 mg/l and 5 mg/l, most preferably of about 3 mg/l. An embodiment is the composition for use according to the invention, wherein the antimicrobial peptide is present in a concentration between 1 mg/l and 10 mg/l, more preferably between 2 mg/l and 5 mg/l, most preferably in a concentration of about 3 mg/l or about 3,3 mg/l. These lower concentrations are in particular useful for maintenance therapy after initial application of a high dose therapy (e.g. with a composition comprising 30 mg/l - 50 mg/l of the antimicrobial peptide) or for prevention of (new) infections.

An embodiment is the composition for use according to the invention, wherein the antimicrobial peptide is present in a concentration between 10 g/l and 100 g/l, more preferably between 20 g/l and 50 g/l, most preferably of about 30 g/l. In another preferred embodiment, a composition for use according to the invention is provided, wherein the antimicrobial peptide is present in a concentration between 10 mg/l and 100 mg/l, more preferably between 20 mg/l and 50 mg/l, most preferably of about 30 mg/l. An embodiment is the composition for use according to the invention, wherein the antimicrobial peptide is present in a concentration between 10 mg/l and 100 mg/l, more preferably between 20 mg/l and 50 mg/l, most preferably in a concentration of about 30 mg/l or about 33 mg/l. These higher concentrations are in particular useful for combating existing infections, in particular when more than 10% of the nail is affected by onychomycosis, and/or if more than one nail is infected by onychomycosis. The infected nail is preferably the nail of a human subject, such as a male human subject, although the human subject can equally well be a female human subject.

The antimicrobial peptide for use according to the invention or the composition for use according to the invention is preferably administered at least once weekly, more preferably at least once every three days, for at least 3 months. Alternatively, the antimicrobial peptide for use according to the invention or composition for use according to the invention is administered at least once every three days, more preferably at least once every two days for at least 2 weeks. More preferably, the peptide or composition is administered at least daily for at least two weeks, more preferably at least 1 month, more preferably for at least 2 months, more preferably for at least 4 months, most preferably for at least 6 months. Preferably, the peptide or composition is administered at least twice a day. More preferably, the peptide or composition is administered at least twice a day for at least 6 months for treatment of very serious onychomycosis infections. The term "very serious onychomycosis infections" relates to existing onychomycosis infections, in particular when more than 10% of the nail is affected by onychomycosis, and/or if more than one nail is infected by onychomycosis. An embodiment is the composition for use according to the invention or the antimicrobial peptide for use according to the invention, wherein the peptide or composition is administered at least once weakly for at least 3 months. An embodiment is the antimicrobial peptide for use according to the invention or the composition for use according to the invention, wherein the peptide or composition is administered at least once every three days.

An embodiment is the antimicrobial peptide for use according to the invention or the composition for use according to the invention, wherein the peptide or composition is administered at least once every three days, more preferably at least once every two days for at least 2 weeks.

An embodiment is the antimicrobial peptide for use according to the invention or the composition for use according to the invention, wherein the peptide or composition is administered at least daily for at least two weeks, more preferably for at least 1 month, more preferably for at least 2 months, more preferably for at least 4 months, most preferably for at least 6 months, such as 6 months to 12 months.

An embodiment is the antimicrobial peptide for use according to the invention or the composition for use according to the invention, wherein the antimicrobial peptide or the composition is administered at least twice a day.

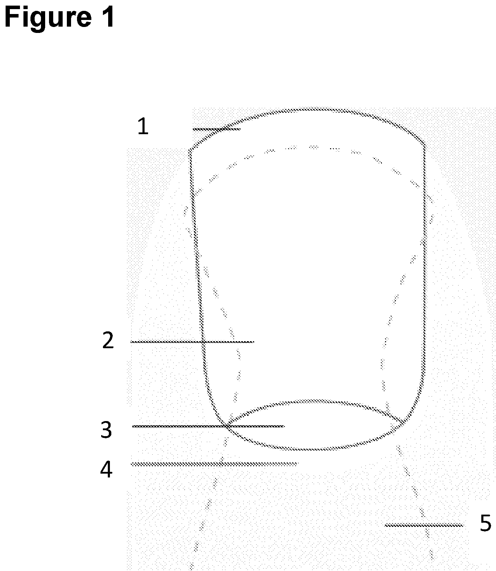

In a preferred embodiment, an antimicrobial peptide for use according to the invention or a composition for use according to the invention is provided, wherein the peptide or composition is applied to the eponychium and/or to the proximal nail fold. Reference is made to Figure 1. In human anatomy, the eponychium, or cuticle, is the thickened layer of skin surrounding fingernails and toenails. It can also be called the medial or proximal nail fold. Its function is to protect the area between the nail and epidermis from exposure to bacteria and other microbes. Growth of nails start in the nail root, hidden under the cuticle. When cells at the root of the nail grow, the new nail cells push out the old nail cells. These old cells flatten and harden, thanks to keratin, a protein made by these cells. The newly formed nail then slides along the nail bed, the flat surface under your nails. Reference is made to Figure 1. Without being bound by theory, it is thought that the antimicrobial peptide of the invention, when applied to the eponychium and/or the proximal nail fold, is incorporated in the newly formed nail and protects the newly formed nail from being infected. The antimicrobial peptide more or less functions as a barrier. When applied regularly, e.g. daily, the antimicrobial peptide will be continuously incorporated into the new nail, thereby effectively protecting the growing nail from being infected by fungi and/or bacteria. Application to the eponychium and/or the proximal fold can be easily performed by dripping a solution comprising the antimicrobial peptide, such as a composition for use according to the invention onto the cuticle. In addition, the antimicrobial peptide may be applied to the lateral nail folds and/or the lateral nail grooves. First, when dripping onto the cuticle, the solution containing the antimicrobial peptide in general will flow lateral from the nail into the lateral nail grooves and, thus, application thereto will in all likelihood occur automatically. Second, application to the lateral folds and/or grooves may have the additional effect of killing fungi and/or bacteria and/or other microbes that have accumulated at the lateral nail grooves or on the lateral nail folds, which helps in clearing the nail from the infection. Some types of onychomycosis, such as paronychia, seen infrequently in older persons, is an acute or chronic nail fold infection that might result in secondary nail plate changes. Acute paronychia, typically induced by trauma, most commonly presents as tender erythematous nail fold swelling of one finger and is usually caused by *Staphylococcus aureus.* Chronic paronychia, on the other hand, manifests as erythematous and swollen nail folds with cuticle loss and secondary changes in the nail plate in the form of multiple transverse ridges. *Candida* species or Gram-negative bacteria are the usual pathogens.

In addition to applying to the cuticle, the grooves and/or the folds, the antimicrobial peptide may be applied to the nail plate, in order to kill any fungi and/or bacteria that are superficially present on the nail plate. Superficial onychomycosis generally presents as black (caused by dematiaceous fungi) or white (caused by dermatophyte species such as *Tmentagrophytes*) patchy discoloration of the nail plate and is in general secondary to fungal invasion of the dorsal surface of the nail plate. Although it cannot be excluded, it is assumed that application of the antimicrobial peptide to the hardened nail plate does not lead to incorporation of the antimicrobial peptide into the nail plate. Nevertheless, clearing the nail from any superficial fungi and/or bacteria and/or other microbes reduces the risk of infection of other nails and/or the risk of infecting (nails of) other people. In a preferred embodiment, therefore, an antimicrobial peptide for use according to the invention or a composition for use according to the invention is provided, wherein the peptide or the composition is applied to the lateral nail folds and/or to the lateral nail grooves and/or to the nail plate. One further anatomical part of the nail that benefits from applying the antimicrobial peptide for use according to the invention is the hyponychium. The hyponychium is the area of epithelium, particularly the thickened portion, underlying the free edge of the nail plate on the nail. This is in particular useful for distal subungual onychomycosis and for lateral subungual onychomycosis. This subtype of onychomycosis, usually caused by dermathophyte fungi, such as *T rubrum,* presents with onycholysis, subungual hyperkeratosis, nail thickening, and discoloration, and is caused by fungal invasion that starts initially at the hyponychium, with progressive proximal spread along the nail bed. In a preferred embodiment, the invention thus provides an antimicrobial peptide for use according to the invention or a composition for use according to the invention, wherein the peptide or the composition is applied to the hyponychium and/or to the proximal part of the nailbed.

An embodiment is the antimicrobial peptide for use according to the invention or the composition for use according to the invention, wherein the peptide or composition is applied to the eponychium and/or to the proximal nail fold.

An embodiment is the antimicrobial peptide for use according to the invention or the composition for use according to the invention, wherein the peptide or composition is applied to the lateral nail folds and/or to the lateral nail grooves and/or to the nail plate.

An embodiment is the antimicrobial peptide for use according to the invention or the composition for use according to the invention, wherein the peptide or composition is applied to the hyponychium and/or to the proximal part of the nailbed.

An antimicrobial peptide as used in the invention, such as the peptide hLF(1-11) [SEQ ID NO: 3], has been shown to be broadly active towards a variety of fungi [2]. Non-limiting examples of fungi that can be treated with an antimicrobial peptide of the invention are, *e.g., Absidia spp* such as *corymbifera, Alternaria spp., Aspergillus spp.* such as *fumigatus, nidulans, niger, versicolor, tamarii, terreus, unguis, sydowii, Botrytis cinerea, Candida spp.* such as *albicans, auris, glabrata, guilliermondii, haemulonii, kefyr, krusei, lusitaniae, metapsilosis, parapsilosis, tropicalis, Chaetomium globosum, Cladosporium spp,* such as *parahalotolerans, trichoides, Cryptococcus spp.* such as *albidus, curvatus, gattii, neoformans, uniguttulatus, Dekkera spp.* such as *bruxellensis, Epidermophyton floccosum, Exophiala dermatitidis, Fonsecaea pedrosoi, Fusarium spp.* such as *moniliforme, oxysporum, solani, Geotrichium spp., Malassezia spp.* such as *furfur, pachydermatis, restricta, globosa,sympodialis, Meyerozyma spp., Microsporum spp.* such as *canis, gypseum, Mucor spp.,Naganishia spp., Nannizia spp.* such as *gypsea, incurvata, otae, Paracoccidioides brasiliensis, Penicillium spp.* such as *digitatum, expansum, italicum, pinophilum, aff. Carbonarius, chrysogenum,, vermiculatum, Phialophora verrucosa, Phoma exigua, Pichia membranifaciens, Rhizoctonia solani, Rhizopus spp., Saccharomyces spp.* such as *cerevisiae, Sclerotinia sclerotiorum, Sclerotium rolfsii, Sporothrix schenckii, Sporotrichium spp., Torula spp., Trichoderma viride, Trichophyton spp.* such as *mentagrophytes, interdigitale, mucoides rubrum, tonsurans, violaceum, Trichosporon spp.* such as *beigelii, cutaneum, Zygosaccharomyces bailii* and *Zygosaccharomyces bisporus,* and broader categories belonging to dermathophyte or dermatomycosis species group.

A peptide as used in the invention has been shown to be further active against a variety of bacteria [2, 3]. Non-limiting examples of bacteria that can be treated with an antimicrobial peptide of the invention are, *inter alia,: Achrobacter spp., Acinetobacter spp.* such as *baumannii, Aciyaetobacter spp., Acrobacter spp., Actinobacillus actinomycetemcomitans, Actinomyces naeslundii, Aeromonas spp., Bacillus spp.* such as *cereus, subtilus, Bacteroides spp., Bronchothrix spp., Burkholderia cepacia, Campylobacter jejuni, Citrobacter spp., Clostridium spp., Deinococcus radiopugnans, Enterococcus spp*. such as *faecalis, Entrobacter cloacae, Escherichia coli, Flavobacterium aquatile, Fusobacterium spp., Hafnia spp., Helicobacter pylori, Klebsiella spp.* such as *pneumoniae, Lactobacillus spp.* such as *caseii, Listeria monocytogenes, Micrococcus spp., Moraxella catarrhalis, Mycobacterium spp.* such as *avium, Neisseria spp., Pasteurella spp., Prevotella spp., Prophyromonas gingivalis., Proteobacterium spp., Proteus spp., Pseudomonas spp.* such as *aeruginosae, Salmonella spp.,* such as *typhimurium, Serratia marcescens, Shewanella putrefaciens, Shigella spp. such as flexneri, sonnei, Staphylococcus spp.* such as *aureus* (including *MRSA), epidermidis, Streptococcus spp.* such as *caseii, lactis, s mitis, mutans,* and *Vibrio cholera.* Onychomycosis is an infection caused by fungi. A bacterial infection involving the nail in general is an infection of the skin around the nail, which is called paronychia. Paronychia can be caused by fungi or bacteria (or both).

In a preferred embodiment, an antimicrobial peptide for use according to the invention or a composition for use according to the invention is provided, wherein the onychomycosis involves an infection with a fungus chosen from the group consisting of: *Absidia spp* such as *corymbifera, Alternaria spp., Aspergillus spp.* such as *fumigatus, nidulans, niger, versicolor, tamarii, terreus, unguis, sydowii, Botrytis cinerea, Candida spp.* such as *albicans, Candida auris, Candida glabrata, Candida guilliermondii, Candida haemulonii, Candida kefyr, Candida krusei, Candida lusitaniae, Candida metapsilosis, Candida parapsilosis, Candida tropicalis, Chaetomium globosum, Cladosporium spp,* such as *parahalotolerans, trichoides, Cryptococcus spp.* such as *albidus, Cryptococcus curvatus, Cryptococcus gattii, Cryptococcus neoformans, Cryptococcus uniguttulatus, Dekkera spp.* such as *bruxellensis, Epidermophyton floccosum, Exophiala dermatitidis, Fonsecaea pedrosoi, Fusarium spp.* such as *moniliforme, oxysporum, solani, Geotrichium spp., Malassezia spp.* such as *furfur, Malassezia pachydermatis, Malassezia restricta, Malassezia globosa,sympodialis, Meyerozyma spp., Microsporum spp.* such as *canis, Microsporum gypseum, Mucor spp.,Naganishia spp., Nannizia spp.* such as *gypsea, Nannizzia incurvata, Nannizzia otae, Paracoccidioides brasiliensis, Penicillium spp.* such as *digitaturn, Penicillium expansum, Penicillium italicum, Penicillium pinophilum, Penicillium spp.* such as aff. *Carbonarius, chrysogenum,, Penicillium vermiculatum, Phialophora verrucosa, Phoma exigua, Pichia membranifaciens, Rhizoctonia solani, Rhizopus spp., Saccharomyces spp.* such as *cerevisiae, Sclerotinia sclerotiorum, Sclerotium rolfsii, Sporothrix schenckii, Sporotrichium spp., Torula spp., Trichoderma viride, Trichophyton spp.* such as *mentagrophytes, interdigitate, mucoides, Trichophyton rubrum, Trichophyton tonsurans, Trichophyton violaceum, Trichosporon spp.* such as *beigelii, Trichosporon cutaneum, Zygosaccharomyces bailii and Zygosaccharomyces bisporus,* and broader categories belonging to dermathophyte or dermatomycosis species group.

In another preferred embodiment, an antimicrobial peptide for use according to the invention or a composition for use according to the invention is provided, wherein the onychomycosis is accompanied by infection with a bacterium chosen from the group, but not exclusive, consisting of: *Achrobacter spp., Acinetobacter spp.* such as *baumannii, Aciyaetobacter spp., Acrobacter spp., Actinobacillus actinomycetemcomitans, Actinomyces naeslundii, Aeromonas spp., Bacillus spp.* such as *cereus, Bacillus subtilus, Bacteroides spp., Bronchothrix spp., Burkholderia cepacia, Campylobacter jejuni, Citrobacter spp., Clostridium spp., Deinococcus radiopugnans, Enterococcus spp.* such as *faecalis, Entrobacter cloacae, Escherichia coli, Flavobacterium aquatile, Fusobacterium spp., Hafnia spp., Helicobacter pylori, Klebsiella spp.* such as *pneumoniae, Lactobacillus spp.* such as *caseii, Listeria monocytogenes, Micrococcus spp., Moraxella catarrhalis, Mycobacterium spp.* such as *avium, Neisseria spp., Pasteurella spp., Prevotella spp., Prophyromonas gingivalis., Proteobacterium spp., Proteus spp., Pseudomonas spp.* such as *aeruginosae, Salmonella spp.,* such as *Salmonella typhimurium, Serratia marcescens, Shewanella putrefaciens, Shigella spp.* such as *flexneri, Shigella sonnei, Staphylococcus spp.* such as *aureus (including MRSA), Staphylococcus epidermidis, Streptococcus spp.* such as *caseii, Streptococcus lactis, Streptococcus mitis, Streptococcus mutans, and Vibrio cholera.*

Preferably, the onychomycosis involves or is accompanied by an infection with *Microsporum spp., Malassezia spp., Epidermophyton spp., Trichophyton spp., Scopulariopsis spp.,* such as *Scopulariopsis brevicaulis, Candida spp.,* such as *Candida albicans* and/or *Staphylococcus spp.* such as *aureus.* These micro-organisms are most commonly involved in onychomycosis or paronychia. An embodiment is the antimicrobial peptide for use according to the invention or the composition for use according to the invention, wherein the onychomycosis involves an infection with *Microsporum spp., Malassezia spp., Epidermophyton spp., Trichophyton spp.,* and/or *Candida spp.* such as *Candida albicans.* An embodiment is the antimicrobial peptide for use according to the invention or the composition for use according to the invention, wherein the onychomycosis involves an infection with at least one of species of *Fusarium spp., Microsporum spp., Malassezia spp., Epidermophyton spp., Trichophyton spp.,* and/or *Candida spp.* such as *Candida albicans* and/or at least one of other fungal species and/or combinations thereof.

Disclosed is a method of treating a patient, preferably a human patient, suffering from onychomycosis, the method comprising administering a therapeutically effective amount of an antimicrobial peptide comprising the sequence RRRRSVQWCA or ACWQVSRRRR, or a functional mutant thereof to the cuticle of a nail affected by onychomycosis.

Disclosed is a method of treating a patient suffering from onychomycosis, the method comprising administering a therapeutically effective amount of an antimicrobial peptide comprising the sequence RRRRSVQWCA or ACWQVSRRRR, or a functional mutant thereof to the cuticle of a nail affected by onychomycosis.

Disclosed is the method wherein the antimicrobial peptide is administered at least daily as an aqueous solution comprising between 1 - 100 g/l of the antimicrobial peptide for at least two weeks.

An embodiment is the antimicrobial peptide comprising the sequence RRRRSVQWCA or ACWQVSRRRR or GRRRRSVQWCA or ACWQVSRRRRG, or a functional mutant thereof, for use in a method according to the invention for the treatment of a patient suffering from onychomycosis, the method comprising or consisting of the step of administering a therapeutically effective amount of the antimicrobial peptide to the cuticle of a nail affected by the onychomycosis, of said patient.

An embodiment is the antimicrobial peptide for use according to the invention, wherein the antimicrobial peptide is administered at least daily as an aqueous solution comprising between 1 mg/l and 100 mg/l of the antimicrobial peptide, for at least two weeks, such as for between 2 weeks and three years, between 4 weeks and 2 years, 6 weeks - 1 year, 12 weeks - 9 months, 16 weeks - 6 months.

The present invention is described with respect to particular embodiments but the invention is not limited thereto but only by the claims. The embodiments of the invention described herein can operate in combination and cooperation, unless specified otherwise.

### EXAMPLES

The minimum inhibitory concentration (MIC) value is defined as the lowest sample concentration that inhibited growth. In the Examples, the MIC90 value is presented, which MIC90 values are determined according to established methods known to the person with the skills in the relevant art. Experiments; 1x10E5 CFU of various strains/ml were incubated for 24-48 h at 37°C in 4x diluted medium of RPMI 1640 with the indicated concentrations of antimicrobial peptide hLF(1-11) [SEQ ID NO: 3]. As controls various strains cells were incubated for 24-48 h at 37°C with no agent (antimicrobial peptide), and subsequently, growth of the microbial organism (e.g. yeast, bacterium) was monitored by using spectrophotometry with a spectrophotometer at 620 nm. Displayed results (see e.g. Table 1-3) are means of at least three independent experiments. ND = Not done. Representative single assay data are listed rather than a range, for clarity reasons and for ease of comparison of peptide efficacy. The MIC was calculated as the lowest antibiotic (antimicrobial peptide) concentration at which growth of the assessed yeast or bacterium (microbe) was inhibited (inhibitory concentration IC90).

To determine the median inhibitory concentration (IC50) value: The percentage (%) of the bacterial (or yeast) growth inhibition is determined as [(Ac-At)/Ac] × 100, where Ac is an average of six replicates of light absorption values at wavelength 510 nm of the negative controls, and At was an average of six replicates of light absorption values at wavelength 510 nm of the samples. The IC50 value is calculated using the linear relation between the inhibitory probability and concentration logarithm. The IC50 value is expressed as the mean of three independent experiments.

The molecular mass of antimicrobial peptide hLF1-11 (GRRRRSVQWCA) [SEQ ID NO: 3] is 1415,6 Da.

Typically, a composition comprising the antimicrobial peptide according to the invention comprises about 20 mg/l - 100 mg/l of the peptide, such as typically 30 mg/l, and 0,01% acetic acid by volume of the composition, wherein the composition essentially consists of the peptide dissolved in water comprising the acetic acid.

### Example 1

### The composition comprising an antimicrobial peptide of the invention and application thereof

The concentration of the antimicrobial peptide in the composition used for the treatment of onychomycosis by human subjects, was 30 mg/l (3% based on the mass of the aqueous solution).

The composition used comprises the antimicrobial peptide hLF1-11 (GRRRRSVQWCA) [SEQ ID NO: 3] and is a clear colorless liquid in a (glass) bottle with a dropper to facilitate application to the nail. The composition does not contain preservations or perfume. The antimicrobial peptide is completely biodegradable.

The composition is for use in the treatment of nails that are affected by a fungal infection (onychomycosis). The use of the composition improves the appearance of discolored and deformed nails. The composition has an immunological effect and provides natural protection against the fungi that can cause onychomycosis. The invention improves the skin structure of the cuticle by changing the micro-flora of the nail and improving the nail surface. The first visible improvements are usually observed after two to four weeks of twice daily application.

Mode of action; the application of the peptides to the cuticle ensures that the peptides are incorporated in the newly formed nail. The infection is pushed away by the newly formed nail and prevents the fungi from spreading to the newly formed nail.

Several human patients observed a positive discoloration, starting at the proximal nail bed. This means that newly formed nail is not (re)infected.

All treated patients who recovered completely, didn't show signs of re-infection. It is, nevertheless, recommended to use a maintenance dose once every 2-3 months. However, results show that treated patients do not show signs of recurrent infection after 4 months or even longer.

The composition is applied twice a day to the affected nail and cuticle, preferably in the morning and in the evening. Hold the tip of the dropper above the nail. Avoid direct contact between the dropper providing the solution and the infected nail to keep liquid clean. Squeeze the tube gently and drop the drop onto the cuticle / proximal fold. It is preferred to cover the whole nail with a thin layer of the solution. In particular cases it is important that the solution is also applied under the edge of the affected nail (on the distal nail bed) e.g. with a cotton swab, in particular when the infection started from there. Allow the nail to dry for a few minutes after application. The treatment duration varies depending on the severity of the infection; in many cases 3-6 months of treatment may be necessary. For optimal results, continue the treatment until a new healthy nail has fully grown. Do not use nail polish during treatment because the antimicrobial peptide may be less effective because of this. Reference is made to Figure 1.

The invention only works locally, that is to say, the applied antimicrobial peptide exerts its antimicrobial activity locally, not systemically, and the antimicrobial peptide or the composition comprising said peptide can therefore be used during pregnancy and lactation. In the case of very serious onychomycosis conditions, a relatively higher concentration is preferred up to concentration 100 mg/L.

### Example 2

### Clinical study for treating onychomycosis

### Study design:

A peptide solution was tested for its treatment effect on onychomycosis in human subjects. The peptide was antimicrobial peptide hLF1-11 (GRRRRSVQWCA) [SEQ ID NO: 3] in water comprising 0,01% by volume acetic acid, based on the total volume of the peptide solution.

Forty two patients are treated with the peptide solution; 20 patients (4 male / 16 female) were treated with a solution comprising a low concentration of 3 mg/L of the peptide (dose twice per day), 10 patients (3 male / 7 female) were treated with the peptide solution with a high concentration of 30 mg/L of the peptide (dose twice per day) and 12 patients (8 male / 4 female) with a combination of high and low concentration (4 weeks 30 mg/L following by 3 mg/L continuing treatment, dose twice per day).

Patients were categorized into groups based on their skin and nail disorders (visible inspection by independent pedicure); low to mild fungal nail problems (treatment group with 3 mg/L for patients in this group), mild to severe fungal nail problems (combination group 30 mg/L - 3 mg/L) and highly infected onychomycosis (30 mg/L group).

Results: 90% of the patients observed an improvement in the appearance of the nail after 2 weeks, 92% observed improvement after 4-10 weeks of treatment. Complete recovery was visible for >98% of the patients after 24 weeks. No exclusions were made. Some patients with severe infections (diabetic / autoimmune related) in the high / low combination treatment group were treated longer with the peptide at high dose. All patients continued the treatment as long as it takes to recover completely from the fungal nail infection (in general, relating to the outgrowth of a complete new nail). Duration of the trial was 6 - 12 months (depending on the infection and the outgrowth of the nail).

After completing the study; samples from the nail (before and aftertreatment) are examined for presence of dermathophyte fungi by culture, by PCR techniques and by matrix assisted laser desorption / ionization -time mass spectrometry (Maldi-Tof MS).

### Double blind study design:

Groups of human patients are treated with a pharmaceutical composition comprising different concentrations of antimicrobial peptide GRRRRSVWCQA [SEQ ID NO: 3] (at a final concentration of 3 mg/L and 30 mg/L). One control group of patients received only the aqueous solution as the placebo (negative control), without the antimicrobial peptide applied to the nails of these patients in the control group.

### Example 3

### Clinical trial: treating fungal nail of human subjects

The anti-fungal activity of the antimicrobial peptide hLF1-11 (GRRRRSVQWCA) [SEQ ID NO: 3] was tested in a clinical trial with human subjects who suffered from at least one nail apparently with onychomycosis.

In the clinical trial a total of 53 patients with different (severe) clinical signs of onychomycosis were included.

305 culture plates where incubated with fungal nail samples retrieved from the patients. 191 out of the 305 cultured fungal nail samples showed no growth of microbial organisms (= 72%); this number corresponds to values published in literature.

15% of the 305 cultured plates were positive, meaning that the plates comprised fungal species (*Trichophyton* and *Candida*) grown during culturing and thus originating from the fungal nails retrieved from the patients in the clinical trial.

A positive effect of the treatment with the antimicrobial peptide, dissolved in an aqueous acetic acid (0,01 volume%) solution was observed for 41 patients (1 placebo, 18 Low concentrate (3,3 mg/L antimicrobial peptide), 22 high concentrate (33,3 mg/L antimicrobial peptide)).

One of the included 8 placebo patients showed signs of improvement of the nail (visual inspection), and probably this was the result of the ingredients in the control placebo solution (Acetic acid and Citric acid). No results were observed for 12 patients (7 placebo patients out of the group of 8 placebo patients, 3 patients (patients A, B, C) treated with antimicrobial peptide at the low concentration and 2 patients (patients D, E) treated with antimicrobial peptide at the high concentration) during 3-5 months treatment of the fungal nail.

For 3 patients A, B, C who were treated with the low concentration of antimicrobial peptide, and for which now clinical improvement was established, it was established that one patient presented with a positive culture (*Candida parapsilosis*). This *Candida* strain was tested for its resistance against the peptide alone; *in vitro* killing assay: MIC90 value of 25 - 50 mg/L was determined for the strain. See also Tables 1-3 in Example 4, here below. Since the MIC90 value was higher than the dose of antimicrobial peptide in the test solution applied onto the nail of the patient, the absence of clinical improvement after treatment with the test solution comprising 3,3 mg/l antimicrobial peptide is likely due to a too low peptide concentration.

For two patients D, E who were treated with a solution comprising the high concentration of the antimicrobial peptide, in their nail samples strains of *Trichophyton interdigitalis* and *Hypercrealis spp.* were determined. Using the antimicrobial peptide in an in *vitro* killing assay for testing the resistance of these strains against the peptide, showed MIC90 values of 50 mg/L. See also Tables 1-3 in Example 4, here below. Since the MIC90 value was higher than the dose of antimicrobial peptide in the test solution applied onto the nail of the patients, the absence of clinical improvement after treatment with the test solution comprising 33 mg/l antimicrobial peptide is likely due to a too low peptide concentration.

In the clinical trial, these three patients A, B, c and two patients D, E were treated with aqueous solutions of the antimicrobial peptide wherein both concentrations used, i.e. low (3,3 mg/L) and high (33,3 mg/L), were thus below the MIC90 values of these tested and identified fungi in the nails of these patients: *Candida parapsilosis,* MIC90 value of 25 - 50 mg/L; *Trichophyton interdigitalis* and *Hypercrealis spp.,* MIC90 values of 50 mg/L.

In conclusion, the clinical trial results obtained with the panel of 53 patients demonstrated a positive effect of the nail treatment with the aqueous solution comprising the antimicrobial peptide, of 88,64%. The two patients D, E who showed no effect upon treatment where subsequently treated with an aqueous solution of the antimicrobial peptide at a concentration of 100 mg/L during the next months. For both patients D, E, a positive effect was established after 3 months of treatment.

Thus, a positive result of 100% for all patients who were included in the clinical trial is obtainable when the concentration of the antimicrobial peptide is 100 mg/L.

Low concentration solution (3,3 mg/L): improvement observed for the patients, after 4 - 7 months. High concentration solution (33,3 mg/L): improvement observed for the patients, after 3-6 months. Visual improvement of the nails were visible for more than 85% of the patients in the clinical trial. Several patients in the clinical trials made the remarks that previously sensitive and/or painful nails became less sensitive during the course of the trial; previous skin problems such as painful skin disappeared, already starting after 2 weeks of treatment; No adverse side effects by using the solution for longer time; Thick nails became thinner and began shining again, during the course of the treatment.

### Example 4

Overview of established inhibitory concentrations of an antimicrobial peptide with regard to a series of microbial organisms: antimicrobial peptide hLF1-11 (GRRRRSVQWCA) [SEQ ID NO: 3].

Tables 1-4 provide an overview of test results after establishing MIC90 values and IC50 values for the indicated yeast strains and bacterial strains.

**TABLE 1. MIC90 values and IC50 values for antimicrobial peptide hLF1-11 (GRRRRSVQWCA) [SEQ ID NO: 3] towards yeast test organisms.**

| **Yeasts** | |
|---|---|
| | |

| **Test organism** | **Inhibitory concentration‡** |
|---|---|
| *Absidia corymbifera* | MIC = 40 µg/ml |
| *Alternatria spp.* | MIC = 16 µM |
| *Aspergillus spp* | MIC = 2,5-12,5 µg/ml |
| *Aspergillus fumigatus* | MIC = 2,5-25 µg/ml |
| *Aspergillus nidulans* | MIC = 2,5-12,5 µg/ml |
| *Aspergillus niger* | MIC = 50 µg/mL |
| *Aspergillus tamarii* | MIC = 2,5-12,5 µg/ml |
| *Aspergillus terreus* | MIC = 2,5-12,5 µg/ml |
| *Aspergillus ungius* | MIC = 3,1-25 µg/ml |
| *Aspergillus sydowii* | MIC = 2,5-12,5 µg/ml |
| *Aspergillus versicolor* | MIC = 10 µg/ml |
| *Botrytis cinerea* | MIC = 16 µM |
| *Candida spp.* | MIC = 12,5-50 µg/ml |
| *C. albicans 90028* | MIC = 12,5 µg/ml |
| *C. albicans AA (LUMC)* | MIC = 25 µg/mL |
| *C. albicans ATCC 10231* | MIC = 25 µg/mL |
| *C. albicans ATCC 90028* | MIC = 12,5 µg/ml |
| *C. albicans CBS-562* | MIC = 25 µg/mL |
| *C. albicans CMC-1968* | MIC = 25 µg/mL |
| *C. albicans YO1-19* | MIC = 12,5 µg/ml |
| *C. albicans (WT)* | MIC = 25 µg/ml |
| *C. auris* | MIC = 12,5-25 µg/ml |
| *C. glabrata spp.* | MIC = 25 µg/mL |
| *C. glabrata (LUMC)* | MIC = 25 µg/mL |
| *C. glabrata CBS-138* | MIC = 25 µg/mL |
| *C. glabrata CMC-1933* | MIC = 25 µg/mL |
| *C. guilliermondii* | MIC = 5-40 µg/ml |
| *C. guilliermondii CBS-2030* | MIC = 50 µg/ml |
| *C. haemulonii* | MIC = 25 µg/mL |
| *C. kefyr* | MIC = 2,5-12,5 µg/ml |
| *C. krusei spp* | MIC = 10-25 µg/ml |
| *C. krusei (LUMC)* | MIC = 25 µg/mL |
| *C. krusei CMC-2002* | MIC = 25 µg/mL |
| *C. lusitaniae CMC-1944* | MIC = 50 µg/mL |
| *C. metapsilosis,* | MIC = 25 µg/mL |
| *C. parapsilosis (LUMC)* | MIC = 12,5 µg/ml |
| *C. parapsilosis ATCC 22019* | MIC = 12,5 µg/ml |
| *C. parapsilosis CBS-604* | MIC = 25 µg/mL |
| *C. parapsilosis CMC-2039* | MIC = 25 µg/mL |
| *C. tropicalis (LUMC)* | MIC = 25 µg/mL |
| *C. tropicalis spp.* | MIC = 12,5-100 µg/mL |
| *C. tropicalis CBS-94* | MIC = 50 µg/mL |
| *C*. *tropicalis CMC-2041* | MIC = 50 µg/mL |
| *C. neoformans* | MMC = 50 µg/ml |
| *C. parapsilosis (LUMC)* | MIC = 12,5 µg/ml |
| *C. parapsilosis ATTC 22019* | MIC = 12,5 µg/ml |
| C. *parapsilosis CBS #57* | MIC = 25-50 µg/mL |
| *C. parapsilosis CBS #61* | MIC = 25-50 µg/mL |
| *C. parapsilosis CBS #7* | MIC = 25-50 µg/mL |
| *C. parapsilosis CBS-604* | MIC = 25 µg/ml |
| *C. parapsilosis CMC-2039* | MIC = 25 µg/ml |
| *C. tropicalis (LUMC)* | MIC = 25 µg/ml |
| *C. tropicalis CBS-94* | MIC = 50 µg/ml |
| *C. tropicalis CMC-2041* | MIC = 50 µg/ml |
| *Cladosporium spp* | MIC = 3,1-12,5 µg/ml |
| *Cladosporium parahalotolerans,* | MIC = 12,5 µg/ml |
| *Cladosporium trichoides* | MIC = 5 µg/ml |
| *Crypt. neoform.* ▲*SKN7 (UMC)* | MIC = 6,3 µg/ml |
| *Crypt. neoform. WT (UMC)* | MIC = 12,5 µg/ml |
| *Cryptococcus albidus* | MIC = 25 µg/ml |
| *Cryptococcus curvatus* | MIC = 9 µg/ml |
| *Cryptococcus gattii* | MIC = 64 µg/mL |
| *Cryptococcus neoformans* | MIC = 12,5-50 µg/mL |
| *Cryptococcus neoformans* ▲*SKN7 (UMC)* | MIC = 6,3 µg/ml |
| *Cryptococcus neoformans WT* | MIC = 12,5 µg/ml |
| *Cryptococcus uniguttulatus* | MIC = 6 µg/ml |
| *Dekkera bruxellensis* | MIC = 3.75-20 µM |
| *Epidermophyton floccosum* | MIC = 0.31-2.5 µg/ml |
| *Exophiala dermatitidis* | MIC = 2 µg/ml |
| *Fonsecaea pedrosoi* | MIC = 5 µg/ml |
| *Fusarium spp* | MIC = 2.5-5 µg/ml |
| *Fusarium moniliforme* | MIC = 2.5-5 µg/ml |
| *Fusarium oxysporum* | MIC = 8-16 µM |
| *Fusarium solani* | MIC = 8-16 µM |
| *Geotrichium spp* | MIC = 50 µg/mL |
| *M. guilliermondii CBS-2030 (Perugia)* | MIC = 50 µg/mL |
| *Malassezia furfur spp.* | MIC = 12.5-50 µg/mL |
| *Malassezia furfur CBS 1878* | MIC = 12.5-50 µg/mL |
| *Malassezia furfur CBS-4172* | MIC = 50 µg/ml |
| *Malassezia furfur CBS 7019* | MIC = 12.5-50 µg/mL |
| *Malassezia furfur CBS-7982* | MIC = 100 µg/ml |
| *Malassezia furfur CBS-9372* | MIC = 50 µg/ml |
| *Malassezia furfur CBS 9595* | MIC = 25-50 µg/mL |
| *Malassezia furfur CBS 14141* | MIC = 12.5-50 µg/mL |
| *Malassezia globosa* | MIC = 12.5-50 µg/mL |
| *Malassezia pachydermatis CBS-1879* | MIC = 50 µg/ml |
| *Malassezia pachydermatis CBS 1880* | MIC = 25-50 µg/mL |
| *Malassezia pachydermatis CBS 1883* | MIC = 25-50 µg/mL |
| *Malassezia restricta CBS 7877* | MIC = 50 µg/mL |
| *Meyerozyma spp.* | MIC = 25-50 µg/mL |
| *Microsporum canis* | MIC = 40 µg/ml |
| *Microsporum gypseum* | MIC = 20-40 µg/ml |
| *Mucor spp* | MIC = 25-50 µg/mL |
| *Nannizia gypsea* | MIC = 30 µg/ml |
| *Nannizzia incurvata* | MIC = 18 µg/ml |
| *Nannizzia otae* | MIC = 60 µg/ml |
| *Paracoccidioides brasiliensis* | MIC = 0.63-1.25 µg/ml |
| *Penicillium spp* | MIC = 12,5-50 µg/ml |
| *Penicillium spp. such as off. Carbonarius* | MIC = 45 µg/ml |
| *Penicillium digitatum* | MIC = 8 µM |
| *Penicillium expansum* | MIC = 16 µM |
| *Penicillium italicum* | MIC = 8 µM |
| *Penicillium pinophilum* | MIC = 45 µg/ml |
| *Penicillium vermiculatum* | MIC = 45 µg/ml |
| *Phialophora verrucosa* | MIC = 5-10 µg/ml |
| *Phoma exigua* | IC50 = 821 µg/mL |
| *Pichia membranifaciens* | MIC = 45 µg/ml |
| *Rhizoctonia solani* | IC50 = 121 µg/mL |
| *Rhizopus spp* | MIC = 25 µg/mL |
| *Saccharomyces cerevisiae* spp | MIC = 0.63-50 µg/ml |
| *Saccharomyces cerivisiae ATCC 2601* | MIC = 25 µg/mL |
| *Saccharomyces cerevisiae ATCC 2602* | MIC = 25 µg/ml |
| *Saccharomyces cerevisiae CMC-520* | MIC = 50 µg/ml |
| *Sclerotinia sclerotiorum* | IC50 = 31 µg/mL |
| *Sclerotium rolfsii* | IC50 = 231 µg/mL |
| *Sporothrix schenckii* | MIC = 2-10 µg/ml |
| *Sporotrichium spp* | MIC = 40 µg/ml |
| *Torula spp* | MIC = 40 µg/ml |
| *Trichoderma viride* | IC50 = 952 µg/mL |
| *Trichophyton spp* | MIC = 6.3-45 µg/ml |
| *Trichophyton interdigitale* | MIC = 6.3-45 µg/ml |
| *Trichophyton mentagrophytes* | MIC = 6.3-45 µg/ml |
| *Trichophyton mucoides* | MIC = 12,5-50 µg/ml |
| *Trichophyton rubrum* | MIC = 13-60 µg/ml |
| *Trichophyton tonsurans* | MIC = 5-40 µg/ml |
| *Trichophyton violaceum* | MIC = 40 µg/ml |
| *Trichosporon beigelii* | MIC = 40 µg/ml |
| *Trichosporon cutaneum* | MIC = 1.25-18 µg/ml |
| *Zygosaccharomyces bailii* | MIC = 8 µM |
| *Zygosaccharomyces bisprous* | MIC = 40 µg/ml |
| ▲ Genetically modified type species ‡ MIC90 values were determined in RPMI 1640 + Glutamax (or supplemented with Tween20), one-fourth strength of the broth as assay medium. | |

**TABLE 2. MIC90 values for antimicrobial peptide hLF1-11 (GRRRRSVQWCA) [SEQ ID NO: 3] towards yeast test organisms commonly related to Onychomycosis in human subjects.**

| **Yeasts** | |
|---|---|
| Group of microbial organisms involved in Onychomycosis | |

| **Test organism** | **Inhibitory concentration‡** |
|---|---|
| *Microsporum spp* | MIC = 25-50 µg/mL |
| *Epidermophyton spp* | MIC = 25-50 µg/mL |
| *Trichophyton spp* | MIC = 6.3-50 µg/ml |
| *Candida albicans spp* | MIC = 25-50 µg/mL |
| *Malassezia spp* | MIC = 12,5-50 µg/mL |
| *Scopulariopsis brevicaulis* | MIC = 25-50 µg/mL |
| *Trichophyton interdigitalis* | MIC = 25-50 µg/mL |
| *Hypercrealis spp* | MIC = 25-50 µg/mL |
| ‡ MIC90 values were determined in RPMI 1640 + Glutamax (or supplemented with Tween20), one-fourth strength of the broth as assay medium. | |

**TABLE 3. MIC90 values for antimicrobial peptide hLF1-11 (GRRRRSVQWCA) [SEQ ID NO: 3] towards bacterium test organisms.**

| **Bacteria** | |
|---|---|
| | |

| **Test organism** | **Inhibitory concentration‡** |
|---|---|
| *Acinetobacter spp.* | MIC = 12,5 µg/ml |
| *A. baumannii LUH6034* | MIC = 12,5 µg/ml |
| *A. baumannii LUH7312* | MIC = 12,5 µg/ml |
| *A. baumannii RUH134* | MIC = 12,5 µg/ml |
| *A. baumannii RUH875* | MIC = 12,5 µg/ml |
| *Achrobacter spp* | MIC = 12,5 µg/ml |
| *Acinetobacter spp* | MIC = 12,5 µg/ml |
| *Aciyaetobacter spp* | MIC = 12,5 µg/ml |
| *Acrobacter spp* | MIC = 12,5 µg/ml |
| *Actinobacillus actinomycetemcomitans* | MIC = 12,5 µg/ml |
| *Actinomyces naeslundii* | MIC = 12,5 µg/ml |
| *Aeromonas spp* | MIC = 12,5 µg/ml |
| *Bacillus cereus* | MIC = 12,5 µg/ml |
| *Bacillus subtilus* | MIC = 12,5 µg/ml |
| *Bacteroides spp* | MIC = 12,5 µg/ml |
| *Bronchothrix spp* | MIC = 12,5 µg/ml |
| *Burkholderia cepacia* | MIC = 12,5 µg/ml |
| *Campylobacter jejuni* | MIC = 12,5 µg/ml |
| *Citrobacter spp* | MIC = 12,5 µg/ml |
| *Clostridium spp* | MIC = 12,5 µg/ml |
| *Deinococcus radiopugnans* | MIC = 12,5 µg/ml |
| *Escherichia coli* | MIC = 6,3 µg/ml |
| *Enterococcus faecalis* | MIC = 6,3 µg/ml |
| *Entrobacter cloacae* | MIC = 6,3 µg/ml |
| *Flavobacterium aquatile* | MIC = 12,5 µg/ml |
| *Fusobacterium spp* | MIC = 12,5 µg/ml |
| *Haemophilus spp* | MIC = 12,5 µg/ml |
| *Hafnia spp* | MIC = 12,5 µg/ml |
| *Helicobacter pylori* | MIC = 12,5-50 µg/ml |
| *Klebsiella spp* | MIC = 12,5 µg/ml |
| *K. pneumoniae L43816* | MIC = 12,5 µg/ml |
| *Listeria monocytogenes* | MIC = 12,5 µg/ml |
| *Micrococcus spp.* | MIC = 12,5 µg/ml |
| *Moraxella catarrhalis* | MIC = 25 µg/ml |
| *Mycobacterium avium* | MIC = 50 µg/ml |
| *Neisseria spp.* | MIC = 12,5 µg/ml |
| *Pseudomonas aeruginosae* | MIC = 12,5 µg/ml |
| *Pasteurella spp.* | MIC = 12,5 µg/ml |
| *Prevotella spp* | MIC = 12,5 µg/ml |
| *Prophyromonas gingivalis* | MIC = 12,5 µg/ml |
| *Proteobacterium spp* | MIC = 12,5 µg/ml |
| *Proteus spp* | MIC = 6,3 µg/ml |
| *S. epidermidis Type 2163* | MIC = 3,1 µg/ml |
| *Salmonella Newport* | MIC = 12,5 µg/ml |
| *Salmonella typhimurium* | MIC = 12,5 µg/ml |
| *Serratia marcescens* | MIC = 12,5 µg/ml |
| *Shewanella putrefaciens* | MIC = 12,5 µg/ml |
| *Shigella flexneri* | MIC = 12,5 µg/ml |
| *Shigella sonnei* | MIC = 12,5 µg/ml |
| *Staphylococcus aureus (MRSA)* | MIC = 1,6 µg/ml |
| *Staphylococcus aureus 10649* | MIC = 1,6 µg/ml |
| *Staphylococcus aureus 29213* | MIC = 3,1 µg/ml |
| *Staphylococcus epidermidis* | MIC = 3,1 µg/ml |
| *Streptococcus caseii* | MIC = 3,1 µg/ml |
| *Streptococcus lactis* | MIC = 3,1 µg/ml |
| *Streptococcus mitis* | MIC = 3,1 µg/ml |
| *Streptococcus mutans* | MIC = 3,1 µg/ml |
| *Streptocococcus mitis 032* | MIC = 6,3 µg/ml |
| *Streptocococcus mitis 033* | MIC = 6,3 µg/ml |
| *Vibrio cholerae* | MIC = 12,5 µg/ml |
| ‡ MIC90 values were determined in RPMI 1640 Glutamax (or supplemented with Tween20), one-fourth strength of the broth as assay medium. | |

| **TABLE 4^{‡a}: Summary of *in vitro* activity of hLF1-11 [SEQ ID NO:3] with unmodified or modified termini, structure relationship.** | | | | | | |
|---|---|---|---|---|---|---|
| | **strain** | *S. aureus*^{b} | *MRSA* 2141 | *S.epidermidis* | *Strep. mitis* ^{c} | *A. baumannii*^{d} |
| **peptide** | | | | | | |
| unmodified N- and C-terminus | | 3,1 | 6,3 | 3,1 | 12,5 | 6,3 |
| acetylated N-terminus | | 3,1 | 3,1 | 6,3 | 12,5 | 6,3 |
| amidated C-terminus | | 1,6 | 1,6 | 3,1 | 3,1 | 3,1 |
| acetylated N-terminus & amidated C-terminus | | 3,1 | 3,1 | 3,1 | 6,3 | 6,3 |
| | | | | | | |

| | **strain** | *P. aeruginosae* | *K.pneumoniae* | *E.coli* | *C.albicans*^{e} | |
|---|---|---|---|---|---|---|
| **peptide** | | | | | | |
| unmodified N- and C-terminus | | 12,5 | 12,5 | 12,5 | 12,5 | |
| acetylated N-terminus | | 12,5 | 12,5 | 6,3 | 12,5 | |
| amidated C-terminus | | 3,1 | 3,1 | 6,3 | 12,5 | |
| acetylated N-terminus & amidated C-terminus | | 12,5 | 6,3 | 3,1 | 12,5 | |
| ‡: Table 4 is adapted in part from: Carlo PJM Brouwer, Luca Roscini, Gianluigi Cardinali, Laura Debora Casagrande Pieranton, Vincent Robert, Mahfuzur Rahman and Mick M Welling, "Structure-Activity Relationship Study of Synthetic Variants Derived from the Highly Potent Human Antimicrobial Peptide hLF(1-11)", ISSN 2578-0190, Cohesive J Microbiol infect Dis. 1(3). CJMI.000512.2018; 10.31031/CJMI.2018.01.000512. Corte4, DOl: | | | | | | |
| a: MIC values were determined in RPMI 1640 Glutamax one-fourth strength of the broth as medium [as outlined in the reference: Cudic M, Condie BA, Weiner DJ, Lysenko ES, Xiang ZQ, et (2002) Development of novel antibacterial peptides that kill resistant isolates. Peptides 23(12): 2083.]. assay 2071-al. | | | | | | |
| b: MIC values for S. *aureus* ATTC 10649 and S. *aureus* ATTC 29213 were equal. | | | | | | |
| c: MIC values for *Streptococcus mitis* 032 and *Streptococcus mitis* 033 were equal. | | | | | | |
| d: MIC values for *Acinetocater baumannii* strains LUH6034, LUH7312, RUH134 and RUH875 comparable. were | | | | | | |
| e: MIC values for *Candida albicans* YO1-19 and *Candida albicans* ATTC 90028 were comparable | | | | | | |
| The media were selected based on an optimal compromise between the growth of individual and peptide activity. The incubation temperature was 37°C and the incubation time was 16-24h. assays were repeated three of four times; here representative single assay data are given rather a range for clarity and ease of comparison of peptide efficacy. strains The than | | | | | | |

### REFERENCES

1. Crich D. Mechanisms of a chemical glycosylation reaction. Acc Chem Res. 2010;43(8):1144-53
2. Brouwer CPJM, Rahman M, Welling MM. Discovery and development of a synthetic peptide derived from lactoferrin for clinical use. Peptides 2011;32(9): 1953-63
3. Nibbering, P.H.; Ravensbergen, E.; Welling, M.M.; van Berkel, L.A.; van Berkel, P.H.; Pauwels, E.K.; Nuijens, J.H. Human lactoferrin and peptides derived from its N terminus are highly effective against infections with antibiotic-resistant bacteria. Infect. Immun. 2001, 69, 1469-1476

## Claims

1. Antimicrobial peptide for use in a method of preventing or treating onychomycosis, wherein the antimicrobial peptide comprises the amino acid sequence RRRRSVQWCA or ACWQVSRRRR or GRRRRSVQWCA or ACWQVSRRRRG, or a functional mutant thereof.

2. Composition comprising an antimicrobial peptide and a pharmaceutically acceptable diluent or excipient, for use in a method of preventing or treating onychomycosis, wherein the antimicrobial peptide comprises the amino acid sequence RRRRSVQWCA or ACWQVSRRRR or GRRRRSVQWCA or ACWQVSRRRRG, or a functional mutant thereof.

3. Composition for use according to claim 2, wherein the antimicrobial peptide is present in a concentration of between 1 mg/l and 500 mg/l, preferably between 2 mg/l and 250 mg/l, more preferably between 3 mg/l and 150 mg/l, most preferably between 3,3 mg/l and 100 mg/l, such as 33 mg/l or 50 mg/l.

4. Composition for use according to claim 2 or 3, wherein the antimicrobial peptide is present in a concentration between 1 mg/l and 10 mg/l, more preferably between 2 mg/l and 5 mg/l, most preferably in a concentration of about 3 mg/l or about 3,3 mg/l.

5. Composition for use according to claim 2 or 3, wherein the antimicrobial peptide is present in a concentration between 10 mg/l and 100 mg/l, more preferably between 20 mg/l and 50 mg/l, most preferably in a concentration of about 30 mg/l or about 33 mg/l.

6. Antimicrobial peptide for use according to claim 1 or composition for use according to any one of claims 2-5, wherein the peptide or composition is administered at least once weakly for at least 3 months.

7. Antimicrobial peptide for use or composition for use according to claim 6, wherein the peptide or composition is administered at least once every three days.

8. Antimicrobial peptide for use according to claim 1 or composition for use according to any one of claims 2-5, wherein the peptide or composition is administered at least once every three days, more preferably at least once every two days for at least 2 weeks.

9. Antimicrobial peptide for use or composition for use according to claim 8, wherein the peptide or composition is administered at least daily for at least two weeks, more preferably for at least 1 month, more preferably for at least 2 months, more preferably for at least 4 months, most preferably for at least 6 months, such as 6 months to 12 months.

10. Antimicrobial peptide for use according to any one of claims 1 or 6 - 9 or composition for use according to any one of claims 2-9, wherein the antimicrobial peptide or the composition is administered at least twice a day.

11. Antimicrobial peptide for use according to any one of claims 1 or 6 - 10 or composition for use according to any one of claims 2 - 10, wherein the peptide or composition is applied to the eponychium and/or to the proximal nail fold and/or to the lateral nail folds and/or to the lateral nail grooves and/or to the nail plate and/or to the hyponychium and/or to the proximal part of the nailbed.

12. Antimicrobial peptide for use according to any one of claims 1 or 6 - **11** or composition for use according to any one of claims 2 - 13, wherein the onychomycosis involves an infection with at least one of species of *Fusarium, Microsporum, Malassezia, Epidermophyton, Trichophyton,* and/or *Candida albicans* and/or at least one of other fungal species and/or combinations thereof.

13. Antimicrobial peptide comprising the sequence RRRRSVQWCA or ACWQVSRRRR or GRRRRSVQWCA or ACWQVSRRRRG, or a functional mutant thereof, for use in a method for the treatment of a patient suffering from onychomycosis, the method comprising or consisting of the step of administering a therapeutically effective amount of the antimicrobial peptide to the cuticle of a nail affected by the onychomycosis, of said patient.

14. Antimicrobial peptide for use according to claim 13, wherein the antimicrobial peptide is administered at least daily as an aqueous solution comprising between 1 mg/l and 100 mg/l of the antimicrobial peptide, for at least two weeks, such as for between two weeks and three years.

15. Antimicrobial peptide for use according to any one of claims 1 or 6 - 14 or composition for use according to any one of claims 2 - 14, wherein the antimicrobial peptide has a modified C-terminus and/or an N-teminus that is modified by acetylation.

## Patentansprüche

1. Antimikrobielles Peptid zur Verwendung in einem Verfahren zur Vorbeugung oder Behandlung von Onychomykose, wobei das antimikrobielle Peptid die Aminosäuresequenz RRRRSVQWCA oder ACWQVSRRRR oder GRRRRSVQWCA oder ACWQVSRRRRG oder eine funktionelle Mutante davon umfasst.

2. Zusammensetzung, umfassend ein antimikrobielles Peptid und ein pharmazeutisch verträgliches Verdünnungsmittel oder Hilfsstoff, zur Verwendung in einem Verfahren zur Vorbeugung oder Behandlung von Onychomykose, wobei das antimikrobielle Peptid die Aminosäuresequenz RRRRSVQWCA oder ACWQVSRRRR oder GRRRRSVQWCA oder ACWQVSRRRRG oder eine funktionelle Mutante davon umfasst.

3. Zusammensetzung zur Verwendung nach Anspruch 2, wobei das antimikrobielle Peptid vorhanden ist in einer Konzentration zwischen 1 mg/l und 500 mg/l, vorzugsweise zwischen 2 mg/l und 250 mg/l, stärker bevorzugt zwischen 3 mg/l und 150 mg/l, am meisten bevorzugt zwischen 3,3 mg/l und 100 mg/l, wie 33 mg/l oder 50 mg/l.

4. Zusammensetzung zur Verwendung nach Anspruch 2 oder 3, wobei das antimikrobielle Peptid vorhanden ist in einer Konzentration zwischen 1 mg/l und 10 mg/l, mehr bevorzugt zwischen 2 mg/l und 5 mg/l, am meisten bevorzugt in einer Konzentration von etwa 3 mg/l oder etwa 3,3 mg/l.

5. Zusammensetzung zur Verwendung nach Anspruch 2 oder 3, wobei das antimikrobielle Peptid vorhanden ist in einer Konzentration zwischen 10 mg/l und 100 mg/l, mehr bevorzugt zwischen 20 mg/l und 50 mg/l, am meisten bevorzugt in einer Konzentration von etwa 30 mg/l oder etwa 33 mg/l.

6. Antimikrobielles Peptid zur Verwendung nach Anspruch 1 oder Zusammensetzung zur Verwendung nach einem der Ansprüche 2-5, wobei das Peptid oder die Zusammensetzung mindestens einmal wöchentlich für mindestens 3 Monate verabreicht wird.

7. Antimikrobielles Peptid zur Verwendung oder Zusammensetzung zur Verwendung nach Anspruch 6, wobei das Peptid oder die Zusammensetzung mindestens einmal alle drei Tage verabreicht wird.

8. Antimikrobielles Peptid zur Verwendung nach Anspruch 1 oder Zusammensetzung zur Verwendung nach einem der Ansprüche 2-5, wobei das Peptid oder die Zusammensetzung mindestens einmal alle drei Tage, stärker bevorzugt mindestens einmal alle zwei Tage für mindestens 2 Wochen verabreicht wird.

9. Antimikrobielles Peptid zur Verwendung oder Zusammensetzung zur Verwendung nach Anspruch 8, wobei das Peptid oder die Zusammensetzung mindestens täglich für mindestens zwei Wochen, stärker bevorzugt für mindestens 1 Monat, stärker bevorzugt für mindestens 2 Monate, stärker bevorzugt für mindestens 4 Monate, am meisten bevorzugt mindestens 6 Monate, wie 6 Monate bis 12 Monate verabreicht wird.

10. Antimikrobielles Peptid zur Verwendung nach einem der Ansprüche 1 oder 6-9 oder Zusammensetzung zur Verwendung nach einem der Ansprüche 2-9, wobei das antimikrobielle Peptid oder die Zusammensetzung mindestens zweimal täglich verabreicht wird.

11. Antimikrobielles Peptid zur Verwendung nach einem der Ansprüche 1 oder 6-10 oder Zusammensetzung zur Verwendung nach einem der Ansprüche 2-10, wobei das Peptid oder die Zusammensetzung aufgetragen wird auf das Eponychium und/oder auf den proximalen Nagelfalz und/oder an den seitlichen Nagelfalzen und/oder an den seitlichen Nagelrillen und/oder an der Nagelplatte und/oder am Hyponychium und/oder am proximalen Teil des Nagelbetts.

12. Antimikrobielles Peptid zur Verwendung nach einem der Ansprüche 1 oder 6 - 11 oder Zusammensetzung zur Verwendung nach einem der Ansprüche 2 - 13, wobei die Onychomykose eine Infektion beinhaltet mit mindestens einer der Spezies Fusarium, Microsporum, Malassezia, Epidermophyton, Trichophyton und/oder Candida albicans und/oder mindestens eine andere Pilzart und/oder Kombinationen davon.

13. Antimikrobielles Peptid, umfassend die Sequenz RRRRSVQWCA oder ACWQVSRRRR oder GRRRRSVQWCA oder ACWQVSRRRRG, oder eine funktionelle Mutante davon, zur Verwendung in einem Verfahren zur Behandlung eines an Onychomykose leidenden Patienten, wobei das Verfahren den Schritt der Verabreichung einer therapeutisch wirksamen Menge umfasst oder daraus besteht des antimikrobiellen Peptids auf die Kutikula eines Nagels des Patienten, der von der Onychomykose betroffen ist.

14. Antimikrobielles Peptid zur Verwendung nach Anspruch 13, wobei das antimikrobielle Peptid mindestens täglich als wässrige Lösung, umfassend zwischen 1 mg/l und 100 mg/l des antimikrobiellen Peptids, für mindestens zwei Wochen, wie z. B. zwischen zwei wochen und drei jahre, verabreicht wird.

15. Antimikrobielles Peptid zur Verwendung nach einem der Ansprüche 1 oder 6-14 oder Zusammensetzung zur Verwendung nach einem der Ansprüche 2-12, wobei das antimikrobielle Peptid einen modifizierten C-Terminus und/oder einen N-Terminus modifiziert durch Acetylierung, aufweist.

## Revendications

1. Peptide antimicrobien destiné à être utilisé dans une méthode de prévention ou de traitement de l'onychomycose, dans lequel le peptide antimicrobien comprend la séquence d'acides aminés RRRRSVQWCA ou ACWQVSRRRR ou GRRRRSVQWCA ou ACWQVSRRRRG, ou un mutant fonctionnel de celle-ci.

2. Composition comprenant un peptide antimicrobien et un diluant ou excipient pharmaceutiquement acceptable, pour une utilisation dans une méthode de prévention ou de traitement de l'onychomycose, dans laquelle le peptide antimicrobien comprend la séquence d'acides aminés RRRRSVQWCA ou ACWQVSRRRR ou GRRRRSVQWCA ou ACWQVSRRRRG, ou un mutant fonctionnel de celle-ci.

3. Composition pour une utilisation selon la revendication 2, dans laquelle le peptide antimicrobien est présent à une concentration comprise entre 1 mg/l et 500 mg/l, de préférence entre 2 mg/l et 250 mg/l, plus préférentiellement entre 3 mg/l et 150 mg/l, de préférence entre 3,3 mg/l et 100 mg/l, comme 33 mg/l ou 50 mg/l.

4. Composition pour une utilisation selon la revendication 2 ou 3, dans laquelle le peptide antimicrobien est présent à une concentration comprise entre 1 mg/l et 10 mg/l, plus préférentiellement entre 2 mg/l et 5 mg/l, de manière préférée entre toutes à une concentration d'environ 3 mg/l ou d'environ 3,3 mg/l.

5. Composition pour une utilisation selon la revendication 2 ou 3, dans laquelle le peptide antimicrobien est présent à une concentration comprise entre 10 mg/l et 100 mg/l, plus préférentiellement entre 20 mg/l et 50 mg/l, de manière préférée entre toutes à une concentration d'environ 30 mg/l ou d'environ 33 mg/l.

6. Peptide antimicrobien à utiliser selon la revendication 1 ou composition à utiliser selon l'une quelconque des revendications 2 à 5, dans lequel le peptide ou la composition est administré au moins une fois par semaine pendant au moins 3 mois.

7. Peptide antimicrobien à utiliser ou composition à utiliser selon la revendication 6, dans lequel le peptide ou la composition est administré au moins une fois tous les trois jours.

8. Peptide antimicrobien à utiliser selon la revendication 1 ou composition à utiliser selon l'une quelconque des revendications 2 à 5, dans lequel le peptide ou la composition est administré au moins une fois tous les trois jours, plus préférablement au moins une fois tous les deux jours pendant au moins 2 semaines.

9. Peptide antimicrobien pour une utilisation ou composition pour une utilisation selon la revendication 8, dans lequel le peptide ou la composition est administré au moins quotidiennement pendant au moins deux semaines, plus préférablement pendant au moins 1 mois, plus préférablement pendant au moins 2 mois, plus préférablement pendant au moins 4 mois, le plus préférablement pendant au moins 6 mois, comme 6 mois à 12 mois.

10. Peptide antimicrobien à utiliser selon l'une quelconque des revendications 1 ou 6 à 9 ou composition à utiliser selon l'une quelconque des revendications 2 à 9, dans lequel le peptide antimicrobien ou la composition est administré au moins deux fois par jour.

11. Peptide antimicrobien à utiliser selon l'une quelconque des revendications 1 ou 6 à 10 ou composition à utiliser selon l'une quelconque des revendications 2 à 10, dans lequel le peptide ou la composition est appliqué sur l'éponychium et/ou sur le pli proximal de l'ongle et /ou aux plis latéraux de l'ongle et/ou aux sillons latéraux de l'ongle et/ou à la plaque unguéale et/ou à l'hyponychium et/ou à la partie proximale du lit de l'ongle.

12. Peptide antimicrobien à utiliser selon l'une quelconque des revendications 1 ou 6 à 11 ou composition à utiliser selon l'une quelconque des revendications 2 à 13, dans lequel l'onychomycose implique une infection par au moins une des espèces de Fusarium, Microsporum, Malassezia, Epidermophyton, Trichophyton et/ou Candida albicans et/ou au moins une autre espèce fongique et/ou leurs combinaisons.

13. Peptide antimicrobien comprenant la séquence RRRRSVQWCA ou ACWQVSRRRR ou GRRRRSVQWCA ou ACWQVSRRRRG, ou un mutant fonctionnel de celui-ci, pour une utilisation dans une méthode de traitement d'un patient souffrant d'onychomycose, la méthode comprenant ou consistant en l'étape d'administration d'une quantité thérapeutiquement efficace du peptide antimicrobien à la cuticule d'un ongle atteint par l'onychomycose, dudit patient.

14. Peptide antimicrobien pour une utilisation selon la revendication 13, dans lequel le peptide antimicrobien est administré au moins quotidiennement sous forme de solution aqueuse comprenant entre 1 mg/l et 100 mg/l du peptide antimicrobien, pendant au moins deux semaines, de préférence entre deux semaines et trois ans.

15. Peptide antimicrobien à utiliser selon l'une quelconque des revendications 1 ou 6 à 14 ou composition à utiliser selon l'une quelconque des revendications 2 à 12, dans lequel le peptide antimicrobien a une extrémité C-terminale modifiée et/ou une extrémité N-terminale qui est modifié par acétylation.
